Europäisches Patentamt

European Patent Office

Office européen des brevets

(19)

(11) Veröffentlichungsnummer : **0 121 718**
**B1**

(12) # EUROPÄISCHE PATENTSCHRIFT

(45) Veröffentlichungstag der Patentschrift :
**20.01.88**

(51) Int. Cl.⁴ : **A 61 F   5/01**

(21) Anmeldenummer : **84102022.5**

(22) Anmeldetag : **27.02.84**

(54) **Orthopädische Hals- und Kopfstütze.**

(30) Priorität : **10.03.83 DE 3308571**

(43) Veröffentlichungstag der Anmeldung :
**17.10.84 Patentblatt 84/42·**

(45) Bekanntmachung des Hinweises auf die Patenterteilung : **20.01.88 Patentblatt 88/03**

(84) Benannte Vertragsstaaten :
**AT BE CH FR GB IT LI LU NL SE**

(56) Entgegenhaltungen :
**US-A- 3 070 090**
**US-A- 3 477 425**
**US-A- 4 205 667**

(73) Patentinhaber : **Auracher, Walter**
**Colmarer Strasse 45**
**D-7000 Stuttgart 40 (DE)**

(72) Erfinder : **Auracher, Walter**
**Colmarer Strasse 45**
**D-7000 Stuttgart 40 (DE)**

(74) Vertreter : **Hoeger, Stellrecht & Partner**
**Uhlandstrasse 14c**
**D-7000 Stuttgart 1 (DE)**

Beschreibung

Die Erfindung betrifft eine orthopädische Hals- und Kopfstütze nach dem Oberbegriff des Patentanspruchs 1.

Bei einer bekannten orthopädischen Hals- und Kopfstütze (US-A-3 070 090) bleibt dann, wenn die Stütze um den Hals eines Patienten gelegt ist, zwischen den einzelnen Polsterelementen praktisch kein Zwischenraum, in den bei Bedarf ein zusätzliches Polsterteil mit entsprechendem Belüftungsabstand eingelegt werden könnte. Außerdem ist ein die Polsterteile verbindendes Band bei dieser Stütze nicht auf der Rückseite der Polsterelemente angeordnet. All dies führt dazu, daß nicht genügend Luft an die Haut eines die Hals- und Kopfstütze tragenden Patienten gelangen kann. Der Patient fühlt sich somit eingeengt und schwitzt stark, so daß auch die Hals- und Kopfstütze feucht wird und im Laufe der Zeit einen unangenehmen Geruch annimmt. Eine vom Arzt verschriebene Stütze dieser Art wird daher häufig gar nicht mehr vom Patienten angelegt.

Bei einer anderen Hals- und Kopfstütze (US-A-3 477 425) sind zwar lösbare Polsterteile vorgesehen, jedoch sind dieser schmaler als ein den Hals des Patienten umschließendes Band ausgebildet, so daß das Band zumindest an seinem unteren Rand unmittelbar am Hals eines Patienten anliegt und Schweißbildung begünstigt. Entsprechendes gilt schließlich auch für eine weitere bekannte Hals- und Kopfstütze (US-A-4 205 667), wo ebenfalls zwischen zwei Polsterelementen praktisch kein Zwischenraum verbleibt.

Es ist Aufgabe der Erfindung, eine Hals- und Kopfstütze nach dem Oberbegriff des Patentanspruchs 1 so auszubilden, daß eine bessere Luftzirkulation, insbesondere zwischen dem Hals des Patienten und den Bändern sowie zwischen den einzelnen Polsterelementen und Polsterteilen ermöglicht und somit ein Feuchtwerden der Polsterelemente infolge Schweißabsonderung im Halsbereich vermieden ist.

Die Aufgabe wird erfindungsgemäß durch die kennzeichnenden Merkmale des Patentanspruchs 1 gelöst.

Durch diese Ausbildung wird erreicht, daß zwischen den separaten Polsterelementen eine Luftzirkulation stattfindet, die sich teilweise auch bis unter die Randbereiche der einzelnen Polsterelemente erstrecken kann. Ein Feuchtwerden der Polsterelemente infolge Schweißabsonderung im Halsbereich ist daher vermieden.

Die nachstehende Beschreibung bevorzugter Ausführungsformen der Erfindung dient im Zusammenhang mit beiliegender Zeichnung der weiteren Erläuterung. Es zeigen :

Fig. 1 eine erste Ausführungsform einer orthopädischen Hals- und Kopfstütze ;

Fig. 2 eine zweite Ausführungsform einer Hals- und Kopfstütze ;

Fig. 3 schematisch eine Draufsicht der an den Hals eines Patienten angelegten Hals- und Kopfstütze aus Fig. 2 ;

Fig. 4 ein zusätzliches Polsterteil für die Hals- und Kopfstütze aus Fig. 1 in Voreransicht und

Fig. 5 das Polsterteil aus Fig. 4 in Rückansicht.

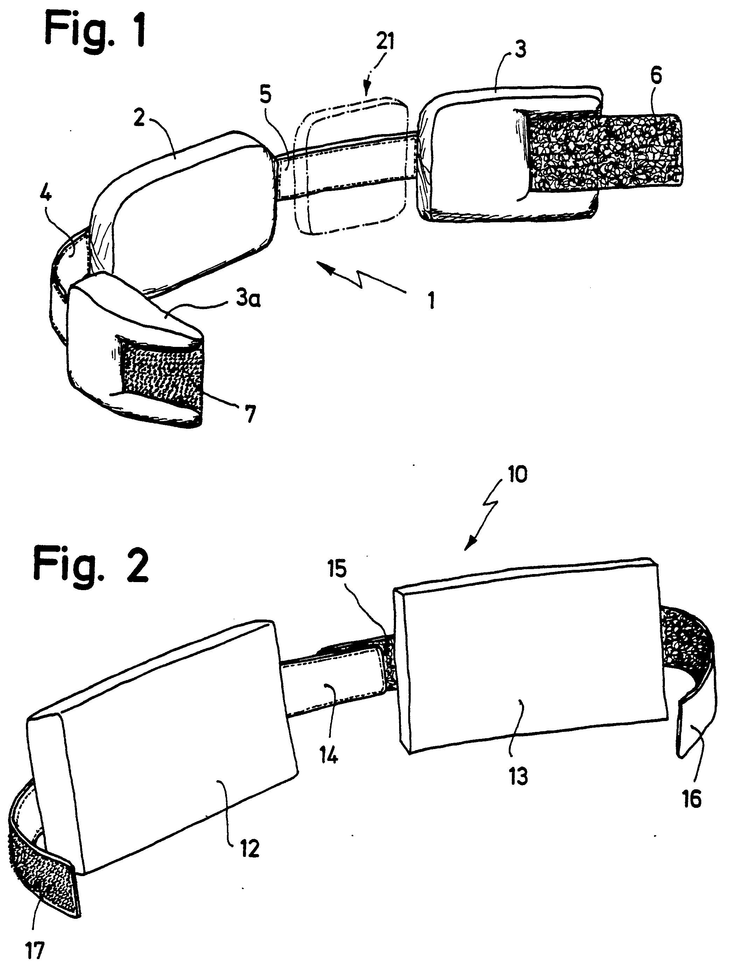

Die in Fig. 1 dargestellte orthopäische Hals- und Kopfstütze 1 weist als wesentliche Bestandteile mehrere, räumlich getrennte Polsterelemente 2, 3 und 3a auf, die durch flexible Bänder 4, 5 beweglich miteinander verbunden sind. Bei angelegter Hals- und Kopfstütze 1 liegen das Polsterelement 2 am Hals eines Patienten unterhalb des Kinns und die beiden Polsterelemente 3, 3a im Nacken, wobei sich die beiden Polsterelemente 3 und 3a gegenseitig, je nach Halsweite des Patienten, mehr oder weniger überlappen. Hierzu sind die Polsterelemente 3, 3a im Überlappungsbereich abgeschrägt oder abgestuft ausgebildet, so daß sie dort insgesamt nicht mehr auftragen, als es der Dicke dieser Polsterelemente im nicht abgeschrägten oder abgestuften Bereich entspricht. Zur gegenseitigen Verbindung der Polsterelemente 3, 3a ist an einem dieser Elemente ein Klettenverschlußband 6 und am andern Polsterelement das zum Band 6 komplementäre Klettenverschlußteil 7 angeordnet. Nach dem Anlegen der Hals- und Kopfstütze 1 rings um den Hals eines Patienten wird das Klettenverschlußband 6 auf den Klettenverschlußteil 7 gepresst und die Stütze somit verschlossen. Dabei liegen die jeweils zwei Polsterelemente miteinander verbindenden Bänder 4, 5 auf der dem Hals des Patienten abgewandten Seite (Außenseite) der Polsterelemente. Hierdurch bildet sich bei angelegter Hals- und Kopfstütze 1 ein Zwischenraum zwischen dem Hals und den Bändern 4, 5, durch den hindurch Luft zirkulieren kann. Da die Bänder 4, 5, wie aus Fig. 1 ersichtlich, auf der Außenseite der Polsterelemente 2, 3, 3a befestigt sind, liegen die der Befestigungsstelle abgewandten Randbereiche nur locker am Hals des Patienten auf, so daß unterhalb dieser Bereiche ebenfalls eine Luftbewegung stattfinden kann. Insgesamt ist somit eine gute Belüftung der Hals- und Kopfstütze 1 gewährleistet, der Patient schwitzt nicht mehr und die Stütze wird nicht feucht.

Die in Fig. 2 dargestellte, abgewandelte Ausführungsform einer Hals- und Kopfstütze 10 umfasst zwei Polsterelemente 12, 13, die durch flexible Klettenverschlußbänder 14, 15 in gegenseitigem Abstand miteinander verbunden sind. Auf den den Klettenverschlußbändern 14, 15 gegenüberliegenden Seiten der Polsterelemente 12, 13 sind weitere Klettenverschlußbänder 16, 17 befestigt. Die Bänder 14, 15, 16 und 17 sind wiederum auf der Außenseite, d. h. auf der dem Hals eines Patienten abgewandten Seite der Polsterelemente 12, 13 befestigt. Durch die beiden Bänder 14, 15 sind die beiden Polsterelemente 12, 13 lösbar miteinander verbunden. Das Polsterelement 12 dient als Kinn-, das Polsterelement 13 als Nackenstütze.

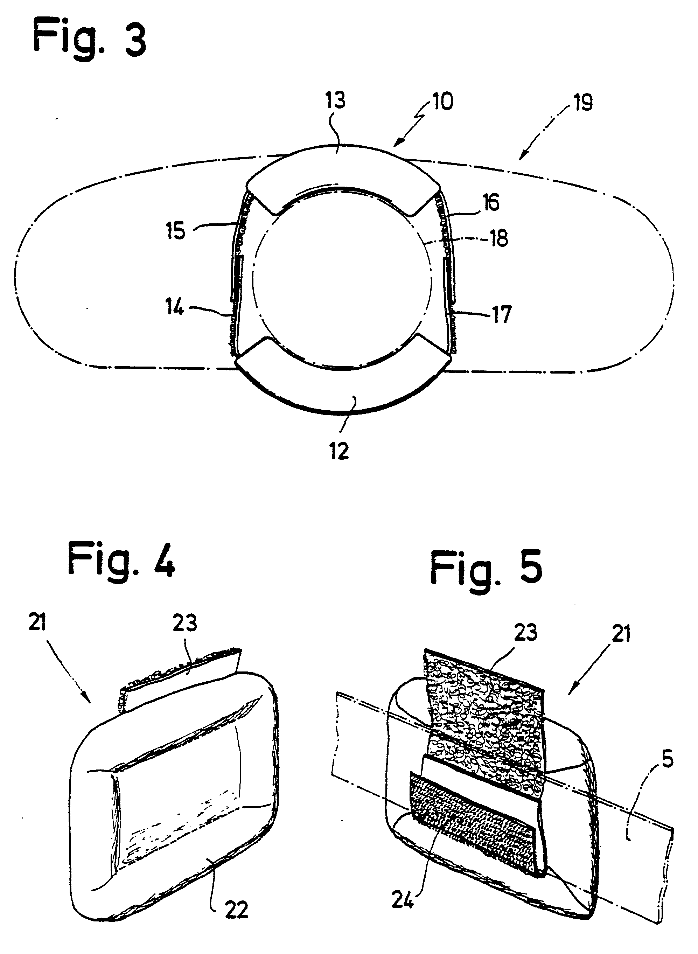

Fig. 3 zeigt schematisch die um den Halsbereich 18 eines Patienten 19 herum angelegte Hals- und Kopfstütze 10 gemäß Fig. 2. Im Gegensatz zur Ausführungsform der Stütze 1 gemäß Fig. 1 ist das im Nacken liegende Polsterelement 13 einstückig. Die Hals- und Kopfstütze 10 wird dementsprechend nicht im Nacken, sondern seitlich, etwa unter dem Ohr des Patienten, durch Verbinden der betreffenden Klettenverschlußbänder, beispielsweise der Bänder 16, 17 geschlossen. Da — wie unmittelbar aus Fig. 3 ersichtlich — die Bänder 14, 15, 16 und 17 an der Außenseite der Polsterelemente 12, 13 befestigt sind, liegen diese Bänder nicht am Hals des Patienten an, und die Hals- und Kopfstütze 10 ist in der im Zusammenhang mit der Ausführungsform gemäß Fig. 1 beschriebenen Weise gut belüftet.

Bei den üblichen Beschwerden, beispielsweise im Zusammenhang mit Halswirbelerkrankungen, reicht es aus, den Kopf lediglich unterhalb des Kinns und im Nackenbereich abzustützen. Eine solche Abstützung ist durch die Hals- und Kopfstützen 1, 10 gemäß Fig. 1 und 2 voll gewährleistet. Falls in Sonderfällen auch eine seitliche Abstützung des Kopfes im Bereich unterhalb des Ohres notwendig ist, so können besondere, zusätzliche Polsterteile an den flexiblen Bändern angeordnet werden, welche die unter dem Kinn und am Nacken liegenden Polsterelemente miteinander verbinden. Dies ist in Fig. 1 im Zusammenhang mit einem zusätzlichen Polsterteil 21 angedeutet, welches im einzelnen in den Figuren 4 und 5 dargestellt ist. Die Vorderseite des Polsterteils 21 kann den aus Fig. 4 ersichtlichen, wulstartigen Randbereich 22 aufweisen. An der Rückseite des Polsterteils 21 (Fig. 5) sind zwei miteinander zusammenwirkende Klettenverschlußbänder 23, 24 befestigt. Diese beiden Klettenverschlußbänder 23, 24 werden um das die beiden Polsterelemente 2, 3 miteinander verbindende Band 5 herum geschlossen. Hierdurch ist das Polsterteil 21 zwischen den Polsterelementen 2, 3 befestigt-vgl. Fig. 1. Da auch zwischen dem Polsterteil 21 und den benachbarten Polsterelementen 2, 3 Zwischenräume vorgesehen sind und die Klettenverschlußbänder 23, 24 an der Rückseite des Polsterteils 21 befestigt sind, ist auch eine gute Belüftung am Hals des Patienten Bereich des Polsterteils 21 gewährleistet.

Die Polsterelemente und Polsterteile 2, 3, 3a, 12, 13 bzw. 21 können in bekannter Weise aus bekanntem Polstermaterial, insbesondere Schaumstoff, bestehen. Geeignet ist insbesondere der unter dem Warenzeichen « Plastazote » vertriebene, verhältnismäßig harte, geschäumte Kunststoff, der weitgehend formbeständig und steif ist und unter Wärme verformt werden kann. Das eigentliche Polstermaterial wird in herkömmlicher Weise mit Trikotschlauchgewebe oder dergleichen, beispielsweise aus Seide oder Baumwolle, überzogen. Auch hautfreundliche Kunststoffolien oder eine Kunststoffbeschichtung kann angewandt werden. Die Polsterelemente und Polsterteile können etwa 5 bis 30 mm dick ausgebildet werden. Ihre die Abstützwirkung bestimmende Höhe kann zwischen 6 und 12 cm bei der Kinnstütze und zwischen 6 und 18 cm bei der Nackenstütze (Polsterelemente 2 bzw. 3) liegen. Die Polsterelemente und Polsterteile können bei Bedarf auch den anatomischen Gegebenheiten des Patienten angepasst werden. So kann beispielsweise insbesondere das als Kinnstütze dienende Polsterelement an seinem oberen Rand leicht konkav gekrümmt verlaufen. Auch andere räumliche Gestaltungen der Polsterelemente als auf der Zeichnung dargestellt sind möglich.

Die Bänder, welche die Polsterelemente miteinander verbinden, können in bekanter Weise als Gummi-, Gurt-, Leder- oder Kunststoffbänder ausgebildet sein. Wesentlich ist, daß sie « halsfern » an den Polsterelementen angeordnet sind.

Die Verschlußelemente zum Verschließen der beschriebenen Hals- und Kopfstütze und zum Befestigen von zusätzlichen Polsterteilen 21 sind vorzugsweise als Klettenverschlußteile ausgebildet. Es können jedoch auch Verschlüsse in Gestalt von Knopf-, Schieber-, Schnallen-, Magnet-, Riemen-, Nestel-, Gurt- und Hakenverschlüsse Anwandung finden. Der Verschluß kann dabei an der Nacken- oder Kinnstütze oder auch seitlich unterhalb des Ohres des Patienten erfolgen.

**Patentansprüche**

1. Orthopädische Hals- und Kopfstütze (1) mit zwei um den Hals (18) eines Patienten (19) zirkulär anlegbaren, mit gegenseitigem Zwischenraum vorgesehenen Polsterelementen (2, 3), von denen das eine (2) im Kinnbereich und das andere (3) im Nackenbereich des Patienten über die gesamte Halshöhe hinweg unmittelbar auf der Haut aufliegt, mit zwei an den Polsterelementen befestigten, flexiblen Bändern (4, 5), die schmäler als die Halshöhe sind und jeweils einen Zwischenraum zwischen den Polsterelementen (2, 3) überbrücken, und mit lösbaren Verschlußelementen (6, 7), dadurch gekennzeichnet, daß zusätzliche Polsterteile (21) vorgesehen sind, die bedarfweise in die Zwischenräume zwischen den Polsterelementen (2, 3) einsetzbar sind, wobei die Breite der zusätzlichen Polsterteile (21) relativ zu den Zwischenräumen zwischen den Polsterelementen (2, 3) so bemessen ist, daß zwischen den zusätzlichen Polsterteilen (21) und den Polsterelementen (2, 3) Belüftungszwischenräume verbleiben, und daß die zusätzlichen Polsterteile (21) mit den diese Zwischenräume überbrückenden Bändern (4, 5) lösbar verbunden sind, wobei diese Bänder (4, 5) auf der dem Hals (18) des Patienten abgewandten Seite (Rückseite) der Polsterelemente (2, 3) bzw. der Polsterteile (21) angeordnet sind, so daß weiterer Belüftungsraum entsteht.

2. Hals- und Kopfstütze nach Anspruch 1, dadurch gekennzeichnet, daß das im Nackenbereich liegende Polsterelement (3) auf zwei abgeschrägten oder abgestuften, sich mehr oder weniger überlappenden, jeweils an einem der Bänder (4, 5) befestigten Teilen (3, 3a) besteht, die durch die Verschlußelemente (6, 7) miteinander verbindbar sind.

3. Hals- und Kopfstütze nach Anspruch 1 oder 2, dadurch gekennzeichnet, daß die zusätzlichen Polsterteile (21) Klettenverschlußteile (23, 24) aufweisen, mit denen sie an einem der Bänder (4, 5) befestigbar sind.

### Claims

1. Orthopaedic neck and head support (1) with padded elements (2, 3) provided with an intermediate space between them and adapted to be fitted circularly around the neck (18) of a patient (19) and of which one (2) rests in the chin area while the other (3) rests in the region of the back of the patient's neck and directly on the skin and over the entire height of the neck, with, fixed on the padded elements, two flexible strips (4, 5) which are narrower than the height of the neck and which in each case bridge an intermediate space between the padded elements (2, 3), and having separable fastening elements (6, 7), characterised in that additional padded parts (21) are provided which can if necessary be inserted into the intermediate spaces between the padded elements (2, 3), the width of the additional padded parts (21) relative to the intermediate spaces between the padded elements (2, 3) being so dimensioned that intermediate ventilation spaces remain between the additional padded parts (21) and the padding elements (2, 3) and in that the additional padded parts (21) are separably connected to the strips (4, 5) bridging these intermediate spaces, the said strips (4, 5) being disposed on that side (the back) of the padded elements (2, 3) or padded parts (21) remote from the patient's neck (18) so that further ventilation space is created.

2. Neck and head support according to Claim 1, characterised in that the padded element (3) located in the region of the back of the neck consists of two angled off or stepped more or less overlapping parts (3, 3a) fixed in each case on one of the strips (4, 5) are adapted to be connected by the fastening elememts (6, 7).

3. Neck and head support according to Claim 1 or 2, characterised in that the additional padded parts (21) comprise velcro fastening parts (23, 24) by which they can be fixed to one of the strips (4, 5).

### Revendications

1. Soutien orthopédique du cou et de la tête (1) ayant deux éléments rembourrés opposés (2, 3) séparés par un intervalle, applicables circulairement autour du cou (18) d'un patient (19), qui s'appliquent directement sur la peau du patient sur toute la hauteur du cou, l'un (2) dans la région du menton et l'autre (3) dans la région de la nuque, avec deux bandes souples (4, 5) fixées aux éléments rembourrés qui sont plus étroites que la hauteur du cou et qui comblent chacune respectivement un des intervalles entre les éléments rembourrés (2, 3), et avec des éléments de fermeture séparables (6, 7), caractérisé en ce que sont prévues des parties rembourrées (21) supplémentaires qui peuvent en cas de besoin être introduites dans les intervalles entre les éléments rembourrés (2, 3), la largeur des parties rembourrées supplémentaires (21) étant dimensionnée par rapport aux intervalles entre les éléments rembourrés (2, 3) de telle façon qu'il subsiste entre les parties rembourrées supplémentaires (21) et les éléments rembourrés (2, 3) des intervalles d'aération, et en ce que les parties rembourrées supplémentaires (21) sont réunies de façon détachable aux bandes (4, 5) comblant ces intervalles, ces bandes (4, 5) étant disposées sur le côté (face arrière) des éléments rembourrés (2, 3) et des parties rembourrées supplémentaires (21), tourné à l'opposé du cou (18) du patient, de sorte qu'un espace supplémentaire d'aération est produit.

2. Soutien du cou et de la tête selon la revendication 1, caractérisé en ce que l'élément rembourré (3) situé dans la région de la nuque est fait de deux parties (3, 3a) à chanfrein ou à gradins, se recouvrant plus ou moins, fixées chacune à l'une des bandes (4, 5) qui peuvent être réunies l'une avec l'autre par les éléments de fermeture (6, 7).

3. Soutien du cou et de la tête selon la revendication 1 ou 2, caractérisé en ce que les parties rembourrées supplémentaires (21) présentent des pièces de fermeture à éléments d'accrochage mutuel (23, 24) à l'aide desquelles elles peuvent être fixées à l'une des bandes (4, 5).

# Fig. 1

# Fig. 2

## Fig. 3

## Fig. 4

## Fig. 5